Europäisches Patentamt

**European Patent Office**

Office européen des brevets

(11) Numéro de publication: **0 061 966**
**B1**

(12) **FASCICULE DE BREVET EUROPEEN**

(45) Date de publication du fascicule du brevet: **10.06.87**

(51) Int. Cl.⁴: **A 61 N 1/36**

(21) Numéro de dépôt: **82400523.5**

(22) Date de dépôt: **23.03.82**

(54) Stimulateur cardiaque comportant une enceinte unique de séparation des moyens générateurs de courant électrique et de production d'impulsions.

(30) Priorité: **27.03.81 FR 8106197**

(43) Date de publication de la demande:
**06.10.82 Bulletin 82/40**

(45) Mention de la délivrance du brevet:
**10.06.87 Bulletin 87/24**

(84) Etats contractants désignés:
**AT BE CH DE GB IT LI LU NL SE**

(56) Documents cités:
**EP-A-0 052 247**
**FR-A-1 524 573**
**FR-A-2 190 403**
**FR-A-2 409 045**
**FR-A-2 466 256**
**US-A-3 190 285**
**US-A-3 739 464**
**US-A-4 010 759**

(73) Titulaire: **CARDIOFRANCE- COMPAGNIE FRANCAISE D'ELECTROCARDIOLOGIE**
**40, Grande Rue**
**F-95450 Commeny (FR)**

(72) Inventeur: **Buffet, Jacques**
**28, avenue Thiers**
**F-93340 Le Raincy (FR)**

(74) Mandataire: **Derambure, Christian**
**Cabinet BUGNION ASSOCIES SARL 116,**
**boulevard Haussmann**
**F-75008 Paris (FR)**

Courier Press, Leamington Spa, England.

## Description

L'invention concerne un stimulateur cardiaque de faible poids et d'encombrement réduit.

On connaît des stimulateurs comportant un boîtier en métal hermétique qui isole les parties constitutives internes des liquides, tels que le sang ou la lymphe qui circulent dans la cavité du corps dans laquelle le stimulateur est implanté. Une pile est disposée dans le boîtier et n'engendre aucun gaz. Elle est constituée par un boîtier de pile dans lequel se trouvent une anode, une cathode et un électrolyte, un des pôles de la pile étant relié au boîtier du stimulateur en métal, ce qui a pour effet de le mettre à la masse. Dans le boîtier du stimulateur se trouve un deuxième boîtier hermétique contenant des circuits électroniques produisant des impulsions transmises soit au coeur soit à un appareil de mesure permettant entre autre de contrôler le fonctionnement du stimulateur. En général, le pile est une pile dont l'anode est en lithium, l'électrolyte étant liquide ou solide. Le pôle positif de la pile est mis à la masse en étant connecté au boîtier en métal. Le pôle négatif de la pile est connecté au circuit électronique, dont la masse est reliée électriquement au boîtier du stimulateur. Ce dernier est en titane, métal compatible avec les liquides du corps. Ainsi, les stimulateurs connus comportent une chambre à circuit électronique, hermétiquement fermée, séparée d'une chambre à pile, hermétiquement fermée.

Le brevet français n° 2190403 décrit un stimulateur cardiaque rechargeable dont le circuit électronique est entouré d'une paroi hermétique qui protège ledit cicuit non seulement contre les effets électromagnétiques dûs à la recharge de l'accumulateur, mais aussi contre d'éventuelles fuites de la solution corrosive de l'accumulateur et dont l'accumulateur rechargeable du type classique est lui-même également entouré de sa propre enveloppe qui l'isole du reste du dispositif.

De même, le brevet français n° 2 409 045 décrit un stimulateur cardiaque dans lequel on met une pile qui comporte sa propre enceinte hermétique et un conformateur d'impulsions classique comportant son propre boîtier.

De tels stimulateurs donnent de bons résultats. Cependant, ils sont relativement lourds, puisque comportant de multiples parois métalliques. On s'oriente actuellement vers une miniaturisation de tels dispositifs dans le but de diminuer la gêne qu'ils pourraient procurer au patient, et de plus pour pouvoir les implanter sur de très jeunes enfants.

Par conséquent, un but de l'invention est de fournir un stimulateur cardiaque de dimensions et d'encombrement relativement réduits, par rapport aux stimulateurs de la technique antérieure. Un autre but de l'invention est de diminuer le coût de fabrication des stimulateurs.

Pour parvenir à ces buts, l'invention propose un stimulateur cardiaque dont les moyens générateurs de courant ne comportent pas d'enceinte hermétique les isolant du boîtier du stimulateur et de l'enceinte de la chambre à circuit électronique. Ainsi, dans un tel stimulateur cardiaque, les moyens générateurs de courant, à savoir une anode, une cathode et un électrolyte, sont placés directement dans le boîtier et il n'existe pas d'enceinte hermétique isolant ces éléments du boîtier, ou les isolant de l'enceinte du circuit électronique. On place dans le boîtier les éléments formant les moyens générateurs de courant et le circuit électronique enfermé dans une enceinte étanche munie d'une borne de sortie pour le raccordement, par exemple à une électrode envoyant des impulsions, et d'une borne pour la connexion électrique avec un des pôles de la pile. On supprime ainsi une paroi métallique et donc le poids du stimulateur en est diminué.

La description suivante, en regard des dessins annexés à titre d'exemples non limitatifs permettrat de mieux comprendre comment l'invention peut être mise en pratique:

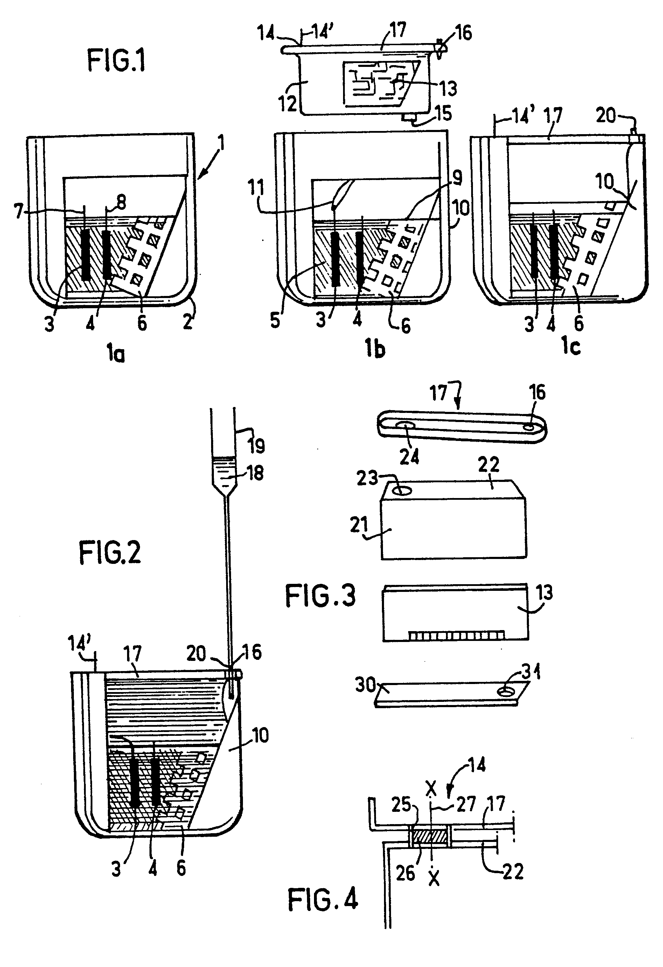

Les figures 1a à 1c sont des schémas montrant les étapes de fabrication d'un stimulateur selon l'invention; la figure 2 est un schéma montrant les étapes supplémentaires de réalisation d'un second mode d'exécution d'un stimulateur selon l'invention; la figure 3 est un schéma représentant en éclaté les parties constitutives du raccord couvercle de boîtier/enceinte du circuit électronique; la figure 4 montre en coupe le raccord couvercle de boîtier/enceinte du circuit électronique.

Le stimulateur 1 comporte un boîtier 2 de forme sensiblement parallélépipédique aplatie. Ce boîtier a environ quatre centimètres de largeur et de longueur et une épaisseur d'environ un centimètre. Il est en matériau conducteur, à savoir un métal compatible avec les humeurs du corps, par exemple du titane. On introduit dans ce boîtier des éléments générateurs de courant continu. Selon un premier mode de réalisation de l'invention ces moyens générateurs de courant sont une anode 3, une cathode 4, un électrolyte solide 5, et éventuellement un séparateur 6, ou autre, moyens qui sont représentés très schématiquement sur les dessins. Ces divers éléments peuvent être mis en place de façon quelconque, par exemple ils peuvent être introduits à force, pour qu'ils restent maintenus en place au fond du boîtier 2. On dispose sur l'anode et la cathode des bornes de sortie 7 et 8. On peut réaliser un joint 9 circulaire situé au-dessus des éléments générateurs de courant. Ce joint 9 peut être étanche, mais dans ce mode de réalisation ce n'est pas nécessaire; par exemple il peut exister un interstice 10 entre la paroi du boîtier 2 et le joint 9. Ce dernier est en matière plastique ou silicone. Par exemple, l'anode peut être en lithium, la cathode peut être constituée d'oxyde de manganèse, l'électrolyte étant solide. Le stimulateur est alors éventuellement transporté dans une atmosphère non humide, c'est-à-dire à degré hygrométrique extrêmement faible, ou sous vide, pour éviter que la vapeur d'eau ne vienne détériorer la surface des éléments générateurs de courant, et plus particulièrement du lithium quand celui-ci constitue l'anode. La borne sortie de l'anode ou de la cathode est reliée à un fil conducteur 11, tandis que l'autre borne de sortie est reliée au boîtier 2 qui est destiné à former masse.

Puis on introduit dans le boîtier 2, au-dessus du joint 9 une enceinte étanche 12 contenant un ou plusieurs circuits électroniques 13 qui sont conçus pour émettre des impulsions soit pour stimuler le coeur, soit pour permettre de contrôler le fonctionnement du stimulateur. L'enceinte 12 est en un matériau métallique. Elle est munie d'une borne de sortie 14 pour le raccordement avec une électrode 14' envoyant les impulsions. L'enceinte 12 est munie en outre d'une borne 15 pour le raccordement électrique avec le fil conducteur 11, donc avec un des pôles anodiques ou cathodiques. La borne sortie 14 est située sur une face de l'enceinte, tandis que la borne 15 est située sur la face opposée. Par ailleurs, sur la face sur laquelle débouche la borne de sortie 14, se trouve éventuellement un orifice 16. En outre, l'enceinte 12 est reliée mécaniquement avec le couvercle 17 du boîtier (voir Figure 1b).

On connecte ensuite la borne 15 avec le fil conducteur 11. Cette borne 15 est reliée électriquement avec une sortie (non représentée) du ou des circuits électroniques tandis qu'une autre sortie des circuits est reliée au boîtier formant masse. Puis on introduit l'enceinte étanche 12 à l'intérieur du boîtier 2. L'orifice 16 vient se situer en regard de l'interstice 10. Enfin, on réalise une soudure bord à bord du couvercle 17 du boîtier et ce dernier. Ainsi, l'autre pôle des éléments générateurs de courant est relié au circuit électronique par l'intermédiaire de cette soudure. Puis on obture l'orifice 16.

On voit que dans ce premier mode de réalisation où l'électrolyte est solide, que l'interstice 10 et l'orifice 16 sont inutiles et qu'on peut les supprimer. On évite ainsi de faire une soudure pour obturer l'orifice 16.

Par contre, selon un second mode de réalisation de l'invention, l'existence de l'interstice 10 et de l'orifice 16 est nécessaire. En effet, les étapes de fabrication du stimulateur sont les mêmes, si ce n'est que l'on n'introduit pas l'électrolyte 5 dans le boîtier avant d'introduire l'enceinte 12 du circuit. Les étapes se déroulent donc de la manière suivante: on introduit l'anode 3 et la cathode 4 dans le fond du boîtier, ainsi que le séparateur 6. On dispose des bornes de sortie 7, 8 et un joint 9 qui comporte obligatoirement pour ce mode de réalisation, un interstice 10. Puis on effectue les connexions entre le fil 11, la borne 15, une des bornes 7, 8, en mettant le boîtier 2 à la masse. On introduit l'enceinte 12 dans le boîtier 2 et on soude le couvercle 17, solidaire de l'enceinte 12, avec le boîtier 2. Le couvercle 17 possède obligatoirement un orifice 16 qui vient se placer en regard de l'interstice 10. Cet orifice est situé dans une partie latérale du couvercle 17, de préférence du côté opposé à la borne de sortie 14 de l'électrode 14'. L'étape supplémentaire consiste à remplir le volume libre laissé entre le boîtier 2 et l'enceinte étanche 12 par un électrolyte liquide 18 à l'aide d'une pipette 19 (voir figure 2). Puis on obture l'orifice 16 comme précédemment. Grâce à ce mode de réalisation, l'électrolyte remplit tout l'espace libre alors qu'il n'en est rien dans les stimulateurs connus, où il y a un risque de fuites de l'électrolyte dans les espaces restant libres. Pour permettre une exécution plus facile du remplissage, on peut prévoir un petit tube 20 traversant l'orifice 16. C'est ce tube qui est soudé à la fin de la fabrication du stimulateur.

Comme on l'a mentionné plus haut, le couvercle 17 du boîtier est solidaire de l'enceinte étanche 12 du ou des circuits électroniques 13, lorsqu'on introduit l'enceinte dans le boîtier 2 (voir figure 1b). En se référant aux figures 3 et 4, on voit que, pour réaliser le raccord entre le couvercle 17 du stimulateur et l'enceinte étanche, on procède de la manière suivante; on place le couvercle 17 sur une des faces d'une boîte 21, de préférence parallélépipédique et ouverte. Sa face 22 de plus grande superficie comporte un trou 23 situé dans une de ses parties latérales extrêmes. Le couvercle du stimulateur possède un trou 24 correspondant au trou 23, situé dans l'une de ses parties latérales extrêmes, et dans l'autre partie opposée latérale, le couvercle 17 comporte un orifice 16 par lequel on peut ultérieurement introduire l'électrolyte liquide. On place le couvercle sur la face 22 de la boîte. Puis on introduit à l'intérieur des trous 23, 24, une pièce cylindrique 25, percée selon son axe de révolution XX, à l'intérieur de laquelle est brasée une pastille 26 circulaire, percée en son centre et traversée par un fil conducteur 27 (qui est la borne 14). Cette pastille est en un matériau isolant par exemple de la céramique telle que de l'alumine frittée. Puis la pièce cylindrique 25 est soudée sur la face 22 de la boîte et sur le couvercle 17. On obtient alors la borne de sortie 14 pour le raccordement de l'électrode 14' émettant des impulsions. On introduit ensuite le ou les circuits électroniques 13 dans la boîte ouverte et l'on raccorde les circuits à la borne 14 et à une borne 15 située dans le trou 31.

On ferme par un couvercle 30 qui forme le fond de l'enceinte étanche 12 ainsi réalisée. La borne 14 est la borne de sortie vers l'électrode, tandis que la borne 15 est la borne de sortie vers un des pôles de la pile.

Bien entendu, la description précédente ne concerne que quelques modes de réalisation de l'invention qui peut comporter d'autres variantes.

**Revendications**

1. Simulateur cardiaque comportant un boitier hermétique métallique (2) constituant un pôle de masse dudit stimulateur, une anode, une cathode et un électrolyte constituant des moyens générateurs de courant électrique continu, une enceinte métallique étanche contenant au moins un circuit électronique (13) de production d'impulsions suceptibles de stimuler le coeur, des liaisons électriques entre lesdits moyens générateurs de courant et ledit circuit électronique stimulateur (13), une borne de sortie (14) d'une électrode (14') reliée audit circuit électronique stimulateur (13), ladite électrode (14') envoyant les impulsions, caractérisé en ce que les moyens générateurs de courant ne comportant pas d'enceinte hermétique les isolant dudit boitier (2) et de l'enceinte (12) dudit circuit électronique stimulateur (13).

2. Stimulateur selon la revendication 1, caractérisé en ce que l'électrolyte des moyens générateurs de courant est un électrolyte solide (5).

3. Stimulateur selon la revendication 1, caractérisé en ce que l'électrolyte des moyens générateurs de courant est un électrolyte liquide (18).

4. Procédé de fabrication d'un stimulateur selon les revendications 1 et 2 comportant les étapes suivantes:
— une anode (3), une cathode (4) et un électrolyte solide (5) sont introduits dans un boitier (2) métallique;
— au moins un circuit électronique (13), contenu dans une enceinte (12) métallique étanche et produisant des impulsions susceptibles de stimuler le coeur, est connecté électriquement d'une part à l'anode ou à la cathode et au boitier formant un pôle de masse et d'autre part à une borne de sortie (14) d'une électrode (14') envoyant les impulsions;
— l'enceinte étanche (12) est introduite dans le boitier métallique (2);
— un couvercle (17) du boitier et le boitier sont soudés bord à bord.

5. Procédé de fabrication d'un stimulateur selon les revendications 1 et 3, comportant les étapes suivantes:
— une anode (3) et une cathode (4) sont introduites dans un boitier (2) métallique;
— au moins un circuit électronique (13), contenu dans une enceinte métallique étanche (12) et produisant des impulsions susceptibles de stimuler le coeur, est connecté électriquement d'une part à la cathode ou à l'anode et au boitier formant un pôle de masse, et d'autre part à une borne de sortie (14) d'une électrode (14') envoyant les impulsions;
— l'enceinte étanche (12) est introduite dans le boitier métallique (2);
— un couvercle (17) du boitier et le boitier sont soudés bord à bord;
— un électrolyte liquide (18) est introduit à travers un orifice (16) du couvercle dans l'espace libre existant entre le boitier (2), l'enceinte étanche (12), l'anode (3) et la cathode (4);
— ledit orifice est obturé.

6. Procédé selon l'une quelconque des revendications 4 et 5, caractérisé en ce qu'après l'introduction de l'anode (3) et de la cathode (4), on place le boitier métallique (2) sous atmosphère sèche.

7. Procédé selon l'une quelconque des revendications 4 et 5, caractérisé en ce qu'après l'introduction de l'anode (3) et de la cathode (4), on place le boitier métallique (2) sous vide.

8. Procédé selon l'une quelconque des revendications 4 à 7, caractérisé en ce que l'on raccorde le couvercle (17) du boitier (2) du stimulateur avec l'enceinte métallique étanche (12) du circuit électronique (13).

9. Procédé selon la revendication 8, caractérisé en ce que le raccord du couvercle du boitier du stimulateur avec l'enceinte étanche du circuit électronique comprend les étapes suivantes:
— on place le couvercle (17) du boitier du stimulateur, comportant un trou (24), sur l'une des faces de plus grande superficie d'une boite (21) métallique ouverte, de préférence parallélépidédique, ladite face comportant un trou (23) situé en regard du trou du boitier;
— on introduit dans les trous une pièce cylindrique (25) munie d'une pastille (26) en matériau isolant;
— on soude ladite pièce cylindrique (25) respectivement sur la boite (21) et sur le couvercle (17) du boitier de stimulateur, en formant ainsi la borne de sortie (14) étanche pour le raccordement d'une électrode emettant des impulsions;
— on introduit le circuit électronique (13) dans la boite ouverte;
— on ferme ladite boite (21) par un couvercle (30) comportant une borne de sortie (15) pour la connexion avec un des pôles anodique et cathodique.

**Patentansprüche**

1. Herzschrittmacher mit einem metallischen, hermetisch dichten Gehäuse (2), das einen Massepol des Schrittmachers bildet, mit einer Anode, einer Kathode und einem Elektrolyten, die die Mittel zur elektrischen Gleichstomerzeugung bilden, mit einem dichten metallischen Behältnis, das zumindest einen elektronischen Schaltkreis (13) zum Erzeugen von zum Stimulieren des Herzens geeigneten Impulsen aufweist, mit elektrischen Verbindungen zwischen dem Stromerzeugermittel und dem elektrischen Stimulationsschaltkreis (13) und mit einer Ausgangsklemme (14) einer Elektrode (14'), wobei die Ausgangsklemme (14) mit dem elektronischen Stimulationsschaltkreis (13) verbunden ist und die Elektrode (14') die Impulse aussendet, dadurch gekennzeichnet, daß das Stromerzeugermittel kein hermetisch dichtes Behältnis, das dieses vom Gehäuse (2) und von der Zelle (12) des elektronischen Stimulationsschaltkreises (13) isoliert, zur Folge hat.

2. Schrittmacher nach Anspruch 1, dadurch gekennzeichnet, daß der Elektrolyt des Stromerzeugermediums ein Festelektrolyt (5) ist.

3. Schrittmacher nach Anspruch 1, dadurch gekennzeichnet, daß der Elektrolyt des Stromerzeugermediums ein flüssiger Elektrolyt (18) ist.

4. Verfahren zum Herstellen eines Schrittmachers nach den Ansprüchen 1 und 2, gekennzeichnet durch die folgenden Schritte:
eine Anode (3), eine Kathode (4) und ein Festelektrolyt (5) werden in das metallische Gehäuse (2) eingesetzt;
zumindest ein elektronischer Schaltkreis (13), der in einem dichten metallischen Behältnis (12) enthalten ist und zum Stimulieren des Herzens geeignete Impulse erzeugt, wird einerseites mit der Anode oder der Kathode und dem Gehäuse, das einen Massepol bildet, und andererseits mit einer Ausgangsklemme (14) einer Elektrode (14'), die die Impulse aussendet, elektrisch verbunden; das dichte Behältnis (12) wird in das metallische Gehäuse (2) eingesetzt;

ein Deckel (17) des Gehäuses und das Gehäuse werden Rand an Rand verschweißt.

5. Verfahren zum Herstellen eines Schrittmachers nach den Ansprüchen 1 und 3, gekennzeichnet durch folgende Schritte:

eine Anode (3) und eine Kathode (4) werden in ein metallisches Gehäuse (2) eingesetzt;

mindestens ein elektronischer Schaltkreis (13), der in einem dichten metallischen Behältnis (12) enthalten ist und zum Stimulieren des Herzens geeignete Impulse erzeugt, wird einerseits mit der Kathode oder der Anode und dem Gehäuse, das einen Massepol bildet, und andererseits mit einer Ausgangsklemme (14) einer Elektrode (14'), die die Impulse aussendet, elektrisch verbunden;

das dichte Behältnis (12) wird in das metallische Gehäuse (2) eingesetzt;

ein Deckel (17) des Gehäses und das Gehäuse werden Rand an Rand verschweißt;

ein Flüssigelektrolyt (18) wird über eine Öffnung (16) im Deckel in den freien Raum zwischen dem Gehäuse (2), des dichten Behältnisses (12), der Anode (3) und der Kathode (4) eingefüllt;

die genannte Öffnung wird verschlossen.

6. Verfahren nach Anspruch 4 oder 5, dadurch gekennzeichnet, daß nach dem Einsetzen der Anode (3) und der Kathode (4) das metallische Gehäuse (2) einer trockenen Atmosphäre ausgesetzt wird.

7. Verfahren nach Anspruch 4 oder 5, dadurch gekennzeichnet, daß nach dem Einsetzen der Anode (3) und der Kathode (4) das metallische Gehäuse (2) unter Vakuum gesetzt wird.

8. Verfahren nach einem der Ansprüche 4 bis 7, dadurch gekennzeichnet, daß der Deckel (17) des Gehäuses (2) des Herzschrittmachers mit dem dichten metallischen Behältnis (12) des elektronischen Schaltkreises (13) verbunden wird.

9. Verfahren nach Anspruch 8, dadurch gekennzeichnet, daß das Verbinden des Deckels des Gehäuses des Schrittmachers mit dem dichten Behältnis des elektronischen Schaltkreises folgende Schritte enthält:

der Deckel (17) des Gehäuses des Schrittmachers, der eine Öffnung (24) aufweist, wird, auf eine der Flächen mit größtem Flächeninhalt eines offenen metallischen, vorzugsweise quaderförmigen Kastens (21) aufgesetzt, dessen Fläche eine Öffnung (23) besitzt, die der Öffnung des Deckels gegenüberliegt;

in diese Öffnungen wird ein zylindrisches Element (25) eingesetzt, das mit einem Plättchen (26) aus isolierendem Material versehen ist;

das zylindrische Element (25) wird am Kasten (21) und am Deckel (17) des Gehäuses des Schrittmachers festgeschweißt, wobei es gleichzeitig die dichte Ausgangsklemme (14) zum Verbinden mit einer Elektrode, die die Impulse aussendet, bildet;

der elektronische Schaltkreis (13) wird in den offenen Kasten eingesetzt;

der Kasten (21) wird mit einem Deckel (30), der eine Ausgangsklemme (15) zum Verbinden mit dem Anoden oder dem Kathoden-Pol trägt, verschlossen.

**Claims**

1. A cardiac stimulator comprising a hermetic metal box (2) operative as earth pole of the stimulator, an anode, a cathode and an electrolyte operative as means for producing d.c., a hermetic metal capsule or the like (12) receiving at least one electronic circuit (13) for producing pulses for stimulating the heart, electrical connections between the current-generating means and the stimulating electronic circuit (13), an output terminal (14) of an electrode (14') connected to the electronic stimulating circuit (13), the electrode (14') transmitting the pulses, characterised in that the current-generating means are devoid of hermetic enclosure insulating them from the box (2) and capsule (12).

2. A stimulator according to claim 1, characterised in that the electrolyte is a solid electrolyte (5).

3. A stimulator according to claim 1, characterised in that the electrolyte is a liquid electrolyte (18).

4. A process for the production of a stimulator according to claims 1 and 2 comprising the following steps:

An anode (3), a cathode (4) and a solid electrolyte (5) are introduced into a metal box (2);

At least one electronic circuit (13) received in a hermetic metal capsule (12) and producing pulses for stumulating the heart is electrically connected, on the one hand, either to the anode or cathode and to the box serving as earth pole and, on the other hand, to an output terminal (14) of the electrode (14') transmitting the pulses;

The capsule (12) is introduced into the box (2), and

A cover (17) for the box is welded thereto edge to edge.

5. A process for the production of a stimulator according to claims 1 and 3 comprising the following steps:

An anode (3) and a cathode (4) are introduced into a metal box (2);

At least one electronic circuit (13) received in a hermetic metal capsule (12) and producing pulses for stimulating the heart is electrically connected, on the one hand, either to the anode or cathode and to the box serving as earth pole and, on the other hand, to an output terminal (14) of an electrode (14') transmitting the pulses;

The capsule (12) is introduced into the box (2);

A cover (17) for the box is welded thereto edge to edge;

A liquid electrolyte (18) is introduced through an aperture (16) in the cover into the gap between the box (2), capsule (12), anode (3) and cathode (4), and

The aperture (16) is sealed.

6. A process according to claim 4 and/or 5, characterised in that after the anode (3) and cathode (4) have been introduced the box (2) is placed in a dry atmosphere.

7. A process according to claim 4 and/or 5, characterised in that after the anode (3) and cathode (4) have been introduced the box (2) is placed in a vacuum.

8. A process according to any of claims 4—7 characterised in that the box cover (17) is connected to the enclosure (12).

9. A process according to claime 8, characterised in that connecting the box cover to the capsule comprises the following steps:

The box cover (17), which is formed with an aperture (24), is placed on one of the larger-area surfaces of an open and preferably parallelepipedic box (21), such surface being formed with an aperture (23) opposite the aperture (24) in the stimulator box (2);

A cylindrical member (25) having a slug or the like (26) of an insulating material is introduced into the apertures (24, 23);

The cylindrical member (25) is welded to the box (21) and to the cover (17) to form the hermetic output terminal (14) for the connection of an electrode transmitting pulses;

The electronic circuit (13) is introduced into the open box (21), and

The same is closed by a cover (30) which has an output terminal (15) for connection to one of the anode and cathode poles.

FIG.1

1a

1b

1c

FIG.2

FIG.3

FIG.4